Europäisches Patentamt

European Patent Office

Office européen des brevets

(11) Publication number: **0 038 156**
A2

(12) **EUROPEAN PATENT APPLICATION**

(21) Application number: **81301482.6**

(22) Date of filing: **06.04.81**

(51) Int. Cl.³: **C 12 N 15/00,** C 12 P 19/34
// C12R1/465, C12P21/02

(30) Priority: **16.04.80 US 140755**

(71) Applicant: **THE UPJOHN COMPANY, 301 Henrietta Street, Kalamazoo, Michigan 49001 (US)**

(43) Date of publication of application: **21.10.81 Bulletin 81/42**

(72) Inventor: **Manis, Jack Jay, 5121 Mapleridge, Portage Michigan (US)**
Inventor: **Stroman, David Womack, 6912 Cromwell, Portage Michigan (US)**

(84) Designated Contracting States: **BE CH DE FR GB IT LI NL SE**

(74) Representative: **Perry, Robert Edward et al, GILL JENNINGS & EVERY 53-64 Chancery Lane, London WC2A 1HN (GB)**

(54) **A plasmid and its microbiological preparation.**

㊴ A novel chemical compound, essentially pure plasmid pUC10, which is obtainable from a biologically pure culture of the microorganism Streptomyces erythreus. Specifically exemplified herein is the obtention of plasmid pUC10 from S. erythreus NRRL 2338. The pUC10 plasmid is useful as a cloning vehicle in recombinant DNA work. For example, using DNA methodology, a desired gene, for example, the insulin gene, can be inserted into pUC10 and the resulting plasmid can then be transformed into a suitable host microbe which, upon culturing, produces the desired insulin.

EP 0 038 156 A2

ACTORUM AG

A PLASMID AND ITS MICROBIOLOGICAL PREPARATION

This invention relates to a novel plasmid and to its microbiological preparation.

The development of plasmid vectors useful for recombinant DNA genetics among microorganisms is well known. The editorial in Science, Vol. 196, April, 1977, gives a good summary of DNA research. This editorial is accompanied by a number of supporting papers in the same issue of Science.

Similar DNA work is currently being done on industrially important microorganisms of the genus Streptomyces. [Bibb, M.J., Ward, J.M., and Hopwood, D.A. 1978. "Transformation of plasmid DNA into Streptomyces at high frequency." Nature 274, 398-400.] Though plasmid DNA's have been detected in several streptomycetes [Huber, M.L.B. and Godfrey, O. 1978. "A general method for lysis of Streptomyces species." Can. J. Microbiol. 24, 631-632.] [Schrempf, H., Bujard, H., Hopwood, D.A. and Goebel, W. 1975. "Isolation of covalently closed circular deoxyribonucleic acid from Streptomyces coelicolor A3(2)." J. Bacteriol. 121, 416-421.] [Umezawa, H. 1977. "Microbial secondary metabolites with potential use in cancer treatment (Plasmid involvement in biosynthesis and compounds)." Biomedicine 26, 236-249.], [Malik, V.S. 1977. Preparative Method for the isolation of super-coiled DNA from a chloramphenicol producing streptomycete. J. Antibiotics 30, 897-899.], only one streptomycete plasmid has been physically isolated and extensively characterized in the literature [Schrempf, supra]. The existence of other plasmids in the genus Streptomyces has been inferred from reported genetic data as follows:

(1) Akagawa, H., Okanishi, M. and Umezawa, H. 1975. "A plasmid involved in chloramphenicol production in Streptomyces venezuelae: Evidence from genetic mapping." J. Gen. Microbiol. 90, 336-346.

(2) Freeman, R.F. and Hopwood, D.A. 1978. "Unstable naturally occurring resistance to antibiotics in _Streptomyces_." J. Gen. Microbiol. 106, 377-381.

(3) Friend, E.J., Warren, M. and Hopwood, D.A. 1978. "Genetic evidence for a plasmid controlling fertility in an industrial strain of _Streptomyces rimosus_." J. Gen. Microbiol. 106, 201-206.

(4) Hopwood, D.A. and Wright, H.M. 1973. "A plasmid of _Streptomyces coelicolor_ carrying a chromosomal locus and its inter-specific transfer." J. Gen. Microbiol. 79, 331-342.

(5) Hotta, K., Okami, Y. and Umezawa, H. 1977. "Elimination of the ability of a kanamycin-producing strain to biosynthesize deoxystreptamine moiety by acriflavine." J. Antibiotics 30, 1146-1149.

(6) Kirby, R., Wright, L.F. and Hopwood, D.A. 1975. "Plasmid-determined antibiotic synthesis and resistance in _Streptomyces coelicolor_." Nature 254, 265-267.

(7) Kirby, R. and Hopwood, D.A. 1977. "Genetic determination of methylenomycin synthesis by the SCPI plasmid of _Streptomyces coelicolor_ A3(2)." J. Gen. Microbiol. 98, 239-252.

(8) Okanishi, M., Ohta, T. and Umezawa, H. 1969. "Possible control of formation of aerial mycelium and antibiotic production in _Streptomyces_ by episomic factors." J. Antibiotics 33, 45-47.

BRIEF SUMMARY OF THE INVENTION

Plasmid pUC10 is obtainable from the known microorganism _Streptomyces erythreus_, NRRL 2338. This plasmid can be obtained from NRRL 2338, and other _S. erythreus_, by growing the culture on a suitable medium, fragmenting the mycelia, incubating the fragmented mycelia, harvesting the culture after a suitable time, and then lysing the mycelia. From this lysate it is possible to isolate essentially pure pUC10.  Plasmid pUC10 is, advantageously, small and has single restriction sites.

pUC10 is characterized by standard characterization
tests which include its molecular weight, approximately
6.8 megadaltons, and endonuclease sites.

pUC10 is useful as a cloning vector in DNA work
wherein desired genes are incorporated into the plasmid,
and the plasmid then transformed into a suitable host.

~~DETAILED DESCRIPTION OF THE INVENTION~~

## The Microorganism

pUC10 is obtainable from the known <u>Streptomyces
erythreus</u>, NRRL 2338.  The microbe is characterized in
U.S. Patent 2,653,899.  This microbe is also available
from the following culture repositories:  CBS 727.72 -
Centraalbureau voor Schimmelcultures, Baarn, Netherlands;
IFO 13426 - Institute for Fermentation, Osaka, Japan.

## Characteristics of pUC10

Molecular Weight:  <u>ca</u>. 6.8 megadaltons.

Restriction Endonuclease Sensitivities:

pUC10 has the following sensitivities to restriction
endonucleases.

### Plasmid pUC10 Sensitivity to Restriction Endonucleases

| | # Cleavage Sites | | |
|---|---|---|---|
| 0 | 1 | 2 | 3 |
| BamHI | BclI | BglII | AvaII |
| HindIII | EcoRI | KpnI | |
| PstI | XhoI | PvuII | |

(Enzyme)

These results were obtained by digestion of pUC10 DNA in the
presence of an excess of restriction endonuclease.  The
number of restriction sites was determined from the number of resolvable fragments in either 0.7 or 1.0% agarose
gels.

pUC10 can be used to create recombinant plasmids
which can be introduced into host bacteria by transformation.  The process of creating recombinant plasmids is
well known in the art.  Such a process comprises cleaving the isolated vector plasmid, e.g., pUC10 at a specific site(s) by means of a restriction endonuclease, for example, BglI, XhoI, or EcoRI. The plasmid, which is a circular DNA molecule, is thus converted into a linear DNA molecule by the enzyme which cuts the two DNA strands at a specific site. Other non-vector DNA is similarly cleaved with the same enzyme. Upon mixing the linear vector or portions thereof and non-vector DNA's, their single-stranded or blunt ends can pair with each other and in the presence of a second enzyme known as polynucleotide ligase can be covalently joined to form a single circle of DNA.

The above procedure also can be used to insert a length of DNA from any plant or animal into pUC10. For example, the DNA which codes for ribosomal RNA in the frog can be mixed with pUC10 DNA that has been cleaved. The resulting circular DNA molecules consist of plasmid pUC10 with an inserted length of frog rDNA.

The recombinant plasmids containing a desired genetic element, prepared by using pUC10, can be introduced into a host organism for expression. Examples of valuable genes which can be inserted into host organisms by the above described process are genes coding for somatostatin, rat proinsulin, and proteases.

The usefulness of plasmid pUC10 is derived from its capacity to function as a plasmid vector in industrially important microorganisms, e.g. Streptomyces. For example, cloning of genetic information from Streptomyces into pUC10 provides a means of increasing the production of commercially important products from these organisms, e.g. antibiotics.

This approach is compared to the concept of cloning genes for antibiotic production into the well characterized Escherichia coli K-12 host-vector system. The E. coli system has the disadvantage that it has been found that genes from some Gram-positive organisms, e.g. Bacillus, do not express well in the Gram-negative E. coli host. Likewise, plasmids from Gram-negative organisms are not maintained in Gram-positive hosts, and Gram-negative genetic information is either expressed poorly or not at all in Gram-positive hosts. This clearly argues for the advantage of a Gram-positive host-vector system and argues the usefulness of plasmid pUC10 in such a system.

In general, the use of a host-vector system to produce a product foreign to that host requires the introduction of the genes for the entire biosynthetic pathway of the product to the new host. As discussed above, this may lead to problems of genetic expression, but may also generate new and/or increased problems in the fermentation of the microorganisms and in the extraction and purification of the product. A perhaps more useful approach is to introduce a plasmid vector, e.g. pUC10 into a host which normally produces the product and clone onto that plasmid the genes for biosynthesis of the product. At the very least, problems of fermentation and product extraction and purification should be minimized. Additionally, in this cloning system it may not be necessary to clone and amplify all the genes of the biosynthetic pathway, but rather it may be necessary only to clone regulatory genes or genes coding for the enzymes that are rate limiting in product biosynthesis. Since pUC10 is a streptomycete plasmid, it is ideally suited for these purposes in the genus Streptomyces. Furthermore, since pUC10 is also a plasmid from a Gram-positive organism, it may serve as a vector in a number of other microorganisms, e.g. Bacillus, Arthrobacter, etc.

Streptomyces erythreus, NRRL 2338, can be grown in an aqueous nutrient medium under submerged aerobic conditions. The organism can be grown in a nutrient medium containing a carbon source, for example, an assimilable carbohydrate, and a nitrogen source, for example, an assimilable nitrogen compound or proteinaceous material. Preferred carbon sources include glucose, brown sugar, sucrose, glycerol, starch, cornstarch, lactose, dextrin, molasses, and the like. Preferred nitrogen sources include cornsteep liquor, yeast, autolyzed brewer's yeast with milk solids, soybean meal, cottonseed meal, cornmeal, milk solids, pancreatic digest of casein, fish meal, distillers' solids, animal peptone liquors, meat and bone scraps, and the like. Combinations of these carbon and nitrogen sources can be used advantageously. Trace metals, for example, zinc, magnesium, manganese, cobalt, iron, and the like, need not be added since tap water and unpurified ingredients are used as components of the medium prior to sterilization of the medium.

The inoculated medium can be incubated at any temperature conducive to satisfactory growth of the microorganism, for example, between about 18° and 35°C., and preferably between about 20° and 32°C. Ordinarily, optimum growth of the microorganism is obtained in about 3 to 15 days. The medium normally remains acidic during the growth cycle. The final pH is dependent, in part, on the buffers present, if any, and in part on the initial pH of the culture medium.

When growth is carried out in large vessels and tanks, it is preferable to use the vegetative form, rather than the spore form, of the microorganism for inoculation to avoid a pronounced lag in the growth of the microorganism and the attendant inefficient utilization of the equipment. Accordingly, it is desirable to produce a vegetative inoculum in a nutrient broth culture by inoculating this broth culture with an aliquot from a soil liquid $N_2$ agar plug, or a slant culture. When a young, active vegetative inoculum has thus been secured, it is transferred aseptically to large vessels or tanks. The medium in which the vegetative inoculum is produced can be the same as, or different from, that utilized for the growth of the microorganism so long as a good growth of the microorganism is obtained.

The following examples are illustrative of the process and products of the subject invention but are not to be construed as limiting. All percentages are by weight and all solvent mixture proportions are by volume unless otherwise noted.

Example 1 - Isolation of Plasmid pUC10 from a Biologically Pure Culture of Streptomyces erythreus, NRRL 2338

The spores from a biologically pure culture of Streptomyces erythreus, NRRL 2338, are inoculated into 10 ml. of the following medium: -1.0% glucose, 0.4% peptone, 0.4% yeast extract, 0.028% MgSO$_4$, 0.2% KH$_2$PO$_4$ and 0.4% K$_2$HPO$_4$.

The medium has previously been sterilized in a 50 ml. Erlenmeyer flask. After inoculation, the flask is incubated at 28-32°C. for about 36 to 48 hours on a Gump or New Brunswick rotary shaker operating at 100-250 rpm. Upon completion of the incubation, the mycelia-broth suspension in the flasks is homogenized under sterile conditions and is then mixed in a sterile 125 ml. Erlenmeyer flask containing 10 ml. of the above medium and also, advantageously, 1% (w/v) glycine. The addition of glycine facilitates the subsequent lysing of the cells. The amounts of glycine in the medium can be varied by routine adjustments with the goal being to facilitate the subsequent lysing of the cells. The flask is then incubated further for another 36 to 48 hours at 28-32°C. on a Gump rotary shaker, as above. After this incubation, the mycelia are separated from the broth by low speed centrifugation, for example, at 6000 x g for 15 minutes at 4°C. and decantation of the supernatant from the mycelial pellet.

The supernatant is discarded and the pellet is resuspended in 1.5 ml. of an isotonic buffer containing ethylenediaminotetraacetic acid (EDTA) and sucrose, e.g. TES buffer [0.03 $\underline{M}$ tris(hydroxymethyl)aminomethane (Tris), 0.005 $\underline{M}$ EDTA and 0.05 $\underline{M}$ NaCl; pH = 8.0] containing 20% (w/v) sucrose. Next, 1.5 ml. of a 5 mg./ml. solution of lysozyme in the same buffer is added and the mixture is incubated at 37°C. for 30 minutes with occasional mixing. Then, 1.5 ml. of 0.25 $\underline{M}$ EDTA (pH = 8.0) is added and this mixture is incubated 15 minutes at 37°C. Subsequently, the cell suspension is lysed by the addition of 2.5 ml. of a lytic mixture, e.g. [1.0% (w/v) Brij-58 (a detergent sold by Pierce Chem. Co., Rockford, Illinois), 0.4% (w/v) deoxycholic acid, 0.05 $\underline{M}$ Tris (pH = 8.0) and 0.06 $\underline{M}$ EDTA] and incubation of this mixture at 37°C. for 20 minutes. The lysate is then sheared by passing it 5-10 times through a 10 ml. pipet. The sheared lysate is then digested with ribonuclease (140 µg/ml.) and pronase (300 µg/ml.) for an additional 20 minutes at 37°C. Alternatively, the cell-lysozyme-EDTA mixture can be digested with ribonuclease and pronase before lysis with a lytic agent such as 2% sodium dodecyl sulfate in water.

This crude lysate material is then mixed with a salt, for example, cesium chloride (preferred), and cesium sulfate, and the intercalating dye ethidium bromide to give a solution of density =1.550. This solution is centrifuged to equilibrium at 145,000 x g. (isopycnic density gradient centrifugation). The covalently closed circular plasmid DNA is then visible in the centrifuge tube under long wave ultraviolet (320 nm.) illumination as a faint fluorescent band below the intensely fluorescent band of linear chromosomal and plasmid DNA's.

Covalently closed circular plasmid DNA is prepared for characterization by removing it from the isopycnic gradients, extracting the ethidium bromide by two treatments with one-third volume of isopropyl alcohol and then dialyzing the aqueous phase against an appropriate buffer, e.g. 0.1 X SSC buffer (0.015 $\underline{M}$ NaCl, 0.0015 $\underline{M}$ sodium acetate; pH = 7.4) to yield essentially pure pUC10.

An estimate of pUC10 molecular weight was obtained by electron microscopy of individual DNA molecules [Kleinschmidt, A.K. (1968). Monolayer techniques in electron microscopy of nucleic acid molecules. In "Method in Enzymology" (S.P. Colowick and N.O. Kaplan, eds.) Vol. 12B, pages 361-377. Academic Press, New York]. Plasmid pUC10 was found to have an average contour length of $3.107 \times 10^{-5}$ meters and a corresponding molecular weight of 6.8 megadaltons.

## Restriction Endonuclease Digestion and Agarose Gel Electrophoresis

Restriction endonucleases were obtained as commercial preparations from Miles Laboratories, Bethesda Research Laboratories, and New England Biolabs. Enzyme digestions were prepared in accordance with the conditions specified by the suppliers using at least a two-fold excess of endonuclease.

The digested samples were applied to 0.7-1% agarose gels and were electrophoresed for 2 hours at a constant applied voltage of 10-15 v/cm of gel height. [Sharp, P.A., Sugden, J. and Sambrook, J. 1973. Detection of two restriction endonuclease activities in Haemophilus parainfluenzae using analytical agarose-ethidium bromide electrophoresis. Biochemistry 12, 3055-3063]. The molecular weights of restriction fragments were determined relative to the standard migration patterns of bacteriophage lambda DNA digested with enzyme HindIII [Murray, K. and Murray, N.E. 1975. Phage lambda receptor chromosomes for DNA fragments made with restriction endonuclease III of Haemophilus influenzae and restriction endonuclease I of Escherichia coli. J. Mol. Biol. 98, 551-564] or EcoRI [Helling, R.B., Goodman, H.M. and Boyer, H.W. 1974. Analysis of endonuclease R·EcoRI fragments of DNA from lambdoid bacteriophages and other viruses by agarose-gel electrophoresis. J. Virology 14, 1235-1244].

The work described herein was all done in conformity with physical and biological containment requirements specified in the NIH Guidelines.

CLAIMS

1. Essentially pure plasmid pUC10 which is characterized by a molecular weight of approximately 6.8 megadaltons, and single restriction endonuclease sites for the enzymes EcoRI, BclI, and XhoI.

2. A process for isolating essentially pure pUC10 from Streptomyces erythreus, which comprises:
   (a) growing S. erythreus on a suitable S. erythreus growth medium until sufficient mycelial growth is obtained;
   (b) fragmenting said mycelia;
   (c) incubating said fragmented mycelia in a suitable growth medium, as above;
   (d) harvesting the culture after a suitable time;
   (e) lysing the harvested mycelia; and
   (f) isolating essentially pure pUC10 from the lysate.

3. A process according to claim 2, wherein essentially pure pUC10 is isolated from S. Erythreus, NRRL 2338.

4. A process according to claim 3, which comprises cultivating Streptomyces erythreus, NRRL 2338, in a nutrient medium at a temperature of about 28-32°C. for about 36 to 48 hours.

5. A process according to any of claims 2 to 4, wherein the fragmented mycelia are incubated in a growth medium containing glycine.